# EUROPEAN PATENT APPLICATION

(11) **EP 4 378 929 A1**
(43) Date of publication of application: **05.06.2024**
(21) Application number: 22210257.6
(22) Date of filing: 29.11.2022
(51) Int. Cl.: C07D 201/00, C07D 231/00, C07F 5/02

(54) **SYNTHESIS OF AROMATIC BORONIC ACIDS**

(71) Applicant: ADAMA MAKHTESHIM LTD., 8410001 Beer Sheva (IL)
(72) Inventor: KISIN-FINFER, Einat, 4531212 Hod Hasharon (IL); FALLEK, Reut, 6745801 Tel Aviv (IL)
(74) Representative: ABG Intellectual Property Law, S.L.

(57) **Abstract**

The invention relates to a method for the preparation of phenylboronic acids of formula (I), or combinations thereof, key intermediates in the preparation of compounds of interest in the agrochemical industry. The process comprises adding a borate over a Grignard reagent.

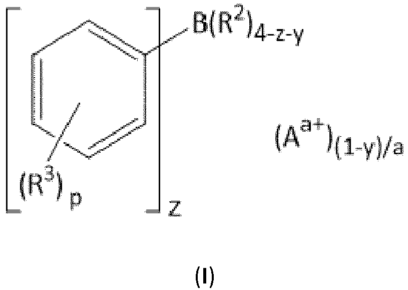

## Description

### FIELD OF THE INVENTION

The present invention relates to the field of synthesis of organic compounds, more specifically to a process for the preparation of aromatic boronic acids, intermediates in the preparation of active ingredients used in the field of agrochemistry.

### BACKGROUND PRIOR ART

The agrochemical industry is always in the search of more efficient processes for the preparation of its active ingredients (Als). The capability of providing economical and clean synthesis of the active ingredients is one of the key factors determining the commercialization of an active ingredient.

Fluxapyroxad, pyraziflumid, bixafen or boscalid are important fungicides. Bixafen was first disclosed in EP 1490342 (Bayer), having the IUPAC name N-(3',4'-dichloro-5-fluoro[1,1'-biphenyl]-2-yl)-3-(difluoromethyl)-1-methyl-1H-pyrazole-4-carboxamide and CAS number 581809-46-3. Fluxapyroxad was first disclosed in EP 1 856 055 (BASF), having the IUPAC name 3-(difluoromethyl)-1-methyl-N-(3',4',5'-trifluoro-[1,1'-biphenyl]-2-yl)-1H-pyrazole-4-carboxamide and CAS number 907204-31-3. Boscalid is disclosed for example in US5589493, having the IUPAC name 2-chloro-N-[2-(4-chlorophenyl)phenyl]pyridine-3-carboxamide and CAS number 188425-85-6. Pyraziflumid is N-[2-(3,4-difluorophenyl)phenyl]-3-(trifluoromethyl)pyrazine-2-carboxamide with CAS number 942515-63-1.

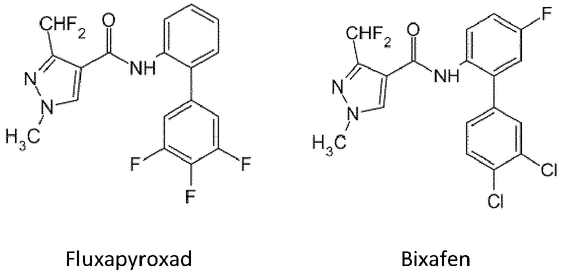

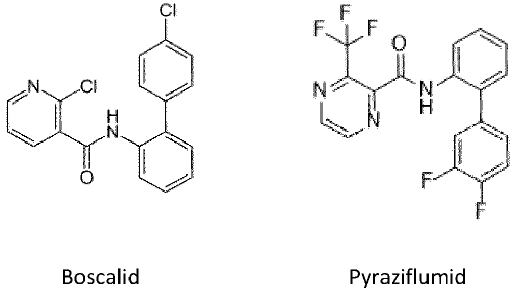

They all belong to the family of the carboxamides and share a substituted *ortho*-biphenylamine (or *ortho*-phenylaniline) residue. One strategy to prepare these *ortho*-biphenylamines requires the condensation between the corresponding aromatic boronic acid and an *ortho*-substituted aniline in the presence of a palladium catalyst. Any improvement in the preparation of the aromatic boronic acids can have a meaningful impact in the preparation of fluxapyroxad, pyraziflumid, bixafen or boscalid.

US2011105766 (BASF SE) describes a process for the palladium catalyzed preparation of some substituted *ortho*-biphenylamines mentioned above. Phenyl boronic acids (i.e. aromatic boronic acids) of formula IVa or of formula IVb are used as starting materials. Section a) of the Preparation Example describes how a phenyl boronic acid (3,4,5-trifluorophenylboronic acid) is prepared. Briefly, the process comprises the preparation of the corresponding Grignard reagent in a first reactor. The so formed Grignard reagent is then metered into a second reactor comprising trimethyl borate at -5°C. That is, is a process wherein the Grignard reagent is added into the phenyl boronic acid. The reported yield was 82%.

US2008183021 (Engel and Oberding) also discloses the production of diphenyl boronic acids of formula (II). Better yields are reported when using only 0.7 equivalents of trialkyl borate (e.g. trimethyl borate) with respect to the Grignard reagent. Example 1 discloses the preparation of di-(4-chlorophenyl)boronic acid by an analogous process wherein the Grignard reagent 4-chlorophenylmagnesium chloride is metered over a solution of trimethyl borate a 11°C. US2008183021 reports a conversion of 87%.

WO2021014437 (Adama) discloses a similar process wherein a Grignard reagent is metered over a solution of trimethyl borate.

### SUMMARY OF THE INVENTION

The inventors have now found that the process for the preparation of aromatic boronic acids can be greatly improved by adding the trialkyl borate over the Grignard reagent. This is the reverse order of addition taught in the prior art documents US2011105766, US2008183021 or WO2021014437.

Thus, a first aspect is a process to prepare a compound of formula (I), or a mixture thereof 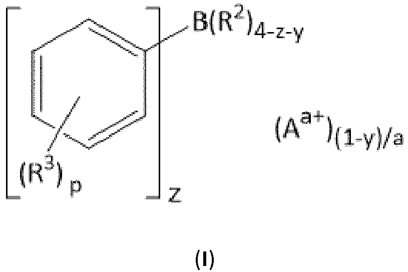 wherein
each R³ is independently selected from the group consisting of chloro, fluoro, C₁-C₆-alkyl, C₁-C₆-haloalkyl, C₁-C₆-alcoxyl and C₁-C₆-haloalcoxyl;
p is an integer selected from the group consisting of 1, 2, 3, 4 and 5
y is an integer selected from 0 or 1;
   wherein,
   when y is 1, then z is 1, 2 or 3;
   when y is 0, then z is an integer selected from 1, 2, 3 or 4, and the compound of formula (I) forms a borate accompanied by a cation A having a charge a+;
   and
each R² is independently selected from the group consisting of hydrogen, halogen, - OH, -OR⁹, and C₁-C₁₀-alkyl, wherein R⁹ is C₁-C₁₀-alkyl or C₆-C₁₂-aryl; or wherein, z being 1 or z being 2 and y being 0, two R² groups together form a bridging group -0-(CH₂)ᵣ-0-, wherein r is 2 or 3, so that said -0-(CH₂)ᵣ-0- group, together with the boron atom, form a 5- or 6-membered ring, where the CH₂ groups are optionally substituted by one or two C₁-C₄-alkyl groups;
the process comprising
a first step comprising the preparation of a compound of formula (II) by contacting a compound of formula (III) with a magnesium source 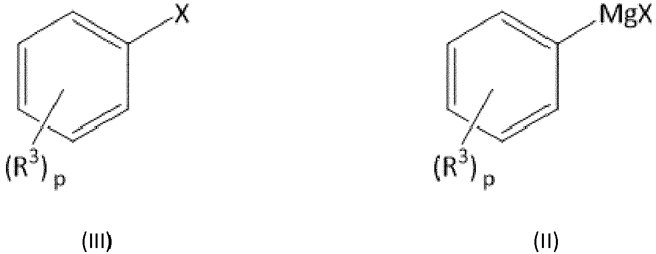 wherein R³ and p are as defined above, and X is a group capable of reacting with magnesium, selected from the group consisting of chloride, bromine, iodide and triflate, for example, bromine, iodide and triflate;
   and
a second step comprising adding over the compound of formula (II) formed in the first step a compound of formula (IV)

   B(R²)₃ (IV)
wherein R² is as defined above.

Considering the literature, it was surprising that changing the order of addition of the reagents would increase the overall yield in the reaction. A Skilled Person would expect that the addition of the Grignard reagent of formula (II) over the compound of formula (IV) provides a more uniform distribution of compounds of formula (I), mostly monoaromatic wherein z is 1. However, the inventors have found that the opposite significantly increases the reaction yield.

The process of the invention provides a more efficient synthesis of compounds of formula (I) or salts thereof, useful intermediates to compounds of interest such as boscalid, pyraziflumid, fluxapyroxad or bixafen. Thus, a second aspect of the invention is a process to process to prepare a compound of formula (V), or a salt thereof 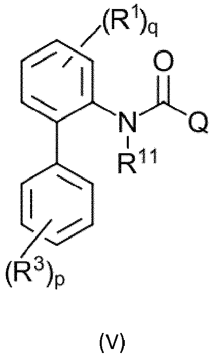 wherein
q is an integer selected from the group consisting of 0, 1, 2, 3 and 4;
p is an integer selected from the group consisting of 1, 2, 3, 4 and 5;
R¹¹ is selected from hydrogen or a nitrogen protecting group;
Q is C₆-C₁₅-aryl or C₃-C₁₅-heteroaryl, optionally substituted with one or more groups selected from the group consisting of halogen, C₁-C₄-alkyl and C₁-C₄-haloalkyl;
each R¹ is independently selected from the group consisting of halogen, -OH, C₁-C₆-alkyl, cyano, C₁-C₆-haloalkyl, C₁-C₆-alcoxyl, and C₁-C₆-haloalcoxyl; and
each R³ is independently selected from the group consisting of chloro, fluoro, C₁-C₆-alkyl, C₁-C₆-haloalkyl, C₁-C₆-alcoxyl and C₁-C₆-haloalcoxyl;
the process comprising
preparing a compound of formula (I) following the process as defined above; and
then transforming said compound of formula (I) in a compound of formula (V).

### DETAILED DESCRIPTION OF THE INVENTION

### Definitions

In the present document the following terms are given the meaning below.

"Halogen" refers in the present document to -F, -Cl, -Br or -I.

"Alkyl" means in the present document a straight or branched hydrocarbon chain radical consisting of carbon and hydrogen atoms, containing no unsaturation, having the number of carbon atoms indicated in each case, for example 1-16 carbon atoms (C₁-C₁₆-), which is attached to the rest of the molecule through a single bond. For example, an alkyl group comprises 1-8 carbon atoms, typically 1-4 carbon atoms. Exemplary alkyl groups can be methyl, ethyl, n-propyl, i-propyl, n-butyl, t-butyl, or n-pentyl.

"Haloalkyl" refers in the present document to an alkyl group that comprises one or more halogen substituents, that is, substituted with at least one of -F, -Cl, -Br or -I. Haloalkyl groups may comprise, for example, 1, 2, 3, 4, 5, 6, 7 or 8 halogen substituents. Haloalkyl groups wherein all positions have been substituted with halogen atoms are also known as perhalo, for example, perfluoro or perchloro substituents. Exemplary haloalkyl groups can be -CH₂F, -CH₂Cl, -CHF₂, - CF₃, -CCl₃, or -CF₂CF₃.

"Cycloalkyl" means in the present document an alkyl group forming a closed ring and attached to the rest of the molecule through a single bond. Cycloalkyl groups can be substituted with other alkyl groups or form more than one ring. Exemplary cycloalkyl groups can be cyclopropyl, 2-mehtylcyclopropyl, cyclobutyl, cyclopentyl, cyclohexyl, 2-mehtylcyclohexyl, 4-mehtylcyclohexyl, cycloheptyl or cyclooctyl.

"Cyano" means in the present document -CN.

"Alcoxyl" means in the present document a radical of the formula -O-alkyl, wherein alkyl has the same meaning as above. Exemplary alkoxyl groups are methoxy, ethoxy or propoxy.

"Aryl" means in the present document an aromatic hydrocarbon radical having the number of carbon atoms indicated in each case, such as phenyl or naphthyl. The aryl radical may be optionally substituted by one, two or three groups selected from the group consisting of halogens, C₁-C₆-alkyl and C₁-C₆-haloalkyl groups.

"Aralkyl" means in the present document an aryl group linked to the rest of the molecule through an alkyl group such as benzyl and phenethyl.

"Heteroaryl" means in the present document a stable 3- to 15- membered cycloalkyl ring system attached to the rest of the molecule through a single bond, wherein one to five carbons atoms of the ring scaffold are replaced by heteroatoms selected from the group consisting of nitrogen, oxygen, and sulphur, and wherein at least one of the rings is aromatic. For the purposes of this invention, the heteroaryl may be for example a monocyclic, bicyclic or tricyclic ring system, which may include fused ring systems; and the nitrogen, carbon or sulphur atoms in the heteroaryl radical may be optionally oxidised; the nitrogen atom may be optionally quaternized. For example, an heteroaryl group can comprise a 4- to 8-membered ring with one or more heteroatoms, for example a 5- or 6-membered ring, wherein one, two or three carbons atoms of the ring scaffold are replaced by heteroatoms selected from the group consisting of nitrogen, oxygen, and sulphur, and wherein at least one of the rings is aromatic. Examples of such heteroaryl groups include, but are not limited to pyridine, pyrazole, pyrazine, benzimidazole, benzothiazole, furan, isothiazole, imidazole, indole, purine, quinoline, thiadiazole.

"Triflate" refers to the -SO₂-CF₃ group, also abbreviated as -OTf.

"Nitrogen protecting group" refers in the present document to a group that blocks an amino group function towards one or more subsequent reactions and that, once said subsequent reactions have taken place, it can be removed under controlled conditions. The amino protecting groups are well known in the art, representative protecting groups are:
- acyls of formula -C(=O)R', such as acetate, benzoate. Pivalate, methoxyacetate, chloroacetate or levulinate;
- carbamates of formula -C(=O)-O-R', such as benzyl carbamate, p-nitrobenzyl carbamate, tert-butyl carbamate, 2,2,2-trichloroethyl carbamate, 2-(trimethylsilyl)ethyl carbamate, allyl carbamate.

In all the above formula R' represents a group selected from the group consisting of alkyl, aryl and aralkyl. Also, different alkyl moieties such as those defined above for R' may be used as amino protecting groups. Additional examples of amino protecting groups can be found in reference books such as Greene and Wuts' "Protective Groups in Organic Synthesis", John Wiley & Sons, Inc., New York, 4th Ed., 2007.

The term "borate" refers to tetrahedral boron anions.

Were indicated, the invention also provides salts of the compounds. For instance, salts of compounds provided herein may be acid addition salts, base addition salts or metallic salts, and they can be synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, non-aqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, ammonium, and organic alkali salts such as, for example, ethylenediamine, ethanolamine, N,N-dialkylenethanolamine, triethanolamine, glucamine and basic aminoacids salts. Examples of the metallic salts include, for example, sodium, potassium, calcium, magnesium, aluminium and lithium salts.

The compounds of formula (I) prepared herein are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon, or ¹⁵N-enriched nitrogen, or ¹⁹F enriched fluorine are within the scope of this invention.

### Process for Preparing Compounds of Formula (I)

The process herein described starts with the reaction between a magnesium source and a compound of formula (III). The magnesium source can be, for example, magnesium metal turnings, magnesium strips, Rieke magnesium (a reactive magnesium powder generated by reduction of a magnesium salt with an alkali metal), sublimed magnesium or magnesium anthracene (typically coordinated with the ether solvent used for its preparation). The most frequent magnesium source used are magnesium metal turnings, due to their availability and lower cost. Magnesium metal is typically covered with a layer of magnesium oxide that reduces reactivity. In order to weaken this passivating layer, activators can be used, such as, Iodine, methyl iodide, or 1,2-dibromoethane.

The reaction requires one equivalent of magnesium for each equivalent of compound of formula (III). In order to ensure that all the compound of formula (III) is consumed, it is typical to add a slight excess of magnesium source. Thus, the proportion in equivalents between the magnesium source and the compound of formula (III) is typically comprised between 1:1 and 2:1, preferably between 1:1 and 1.5:1, preferably between 1:1 and 1.3:1. The reaction is usually executed as a typical Grignard reaction. Typically, the magnesium source is placed in solvent and then a small amount of compound of formula (III) is added in order to initiate the reaction. Once the reaction has been activated (shown by an increase in temperature), the rest of the compound of formula (III) is added, usually metered over time. Once all the compound of formula (III) has been added, if necessary, the reaction is allowed to proceed to completion. The temperature can be raised, for example, to a temperature comprised between 20°C and 120°C, preferably between 40°C and 100°C.

The so formed Grignard reagent of formula (II) is then contacted with the borane of formula (IV). The inventors have found that metering the borane of formula (IV) over the Grignard reagent of formula (II) surprisingly improves the yield. Thus, in the present process the borane is added over the Grignard reagent. Additionally, the operation is easier. Typically, the mixture containing the Grignard reagent of formula (II) is cooled before adding the borane of formula (IV) to control exothermicity, for example, at a temperature below 30°C, preferably below 20°C, typically at a temperature comprised between -20°C and 15°C, for example between -10°C and 10°C. Then, once all or part of the borane of formula (IV) has been added, it is typically possible to allow the reaction to proceed at room temperature.

The stoichiometry of the reaction is again 1 to 1 with respect to the initial compound of formula (III), but some excess of the compound of formula (IV) is typically used to ensure complete consumption of the Grignard reagent of formula (II). Thus, the typical proportion between the compound of formula (IV) and the compound of formula (III) is comprised between 1:1 and 2:1, preferably between 1:1 and 1.5:1, preferably between 1:1 and 1.3:1. In the compound of formula (IV), each R² can be independently selected from a C₁-C₁₀-alkyl group, preferably a C₁-C₄-alkyl group, preferably all R² groups in the compound of formula (IV) are the same and are selected from a C₁-C₄-alkyl group.

The process described herein is preferably carried out in an inert atmosphere. The reaction time depends on the specific conditions (temperature of reaction, solvent used), but it is typically comprised between 1 minute and 10 hours to form the Grignard reagent of formula (II), for example, between 0.1 hours and 5 hours. The reaction between the compound of formula (II) and the borate of formula (IV) typically between 1 minute and 10 hours, for example between 0.1 hours and 5 hours.

After completion, the reaction mixture may require a work-up and isolation of the compound of the formula (I) in a customary manner. The solvents can be removed, for example, under reduced pressure. Preferably, however, the work-up is done by adding aqueous acid (e.g. hydrochloric acid, hydrobromic acid, hidrofluoric acid, sulfuric acid, nitric acid, and phosphoric acid) to induce hydrolysis and stop the reaction, followed by separation of the phases, followed by optional additional washes with water. The hydrolysis may require some time, for example between 1 and 300 minutes, and/or some temperature, for example, between 20°C and 100°C or between 25C° and 80°C.

When preparing the compounds of formula (I) it must be considered that it is frequent to obtain mixtures of different species of compounds of formula (I). For example, boronic acid (i.e. a compound of formula (I) wherein y is 1 and all R² groups are -OH) may be the main compound of formula (I) obtained, but it can be accompanied by a mixture of further compounds of formula (I) in different proportions wherein z is in each case 1, 2 or 3, all being potentially useful in subsequent reactions.

In the compounds of formula (I) z can be 1, 2 or 3 when y is 1, thus defining the number of phenyl groups attached to the boron group. For example, the compounds of formula (I) wherein y is 1, typically comprise species wherein z is 1, preferably defining a boronic acid (both R² are -OH) or a derivative thereof wherein at least one R² is different from -OH. This boronic acids can come in mixtures with the corresponding compounds of formula (I) wherein z is 2 or 3. It is preferred that all R² groups of the compound of formula (I) are -OH. It should also be taken into consideration that such compounds can form trimers associated through non-covalent intermolecular forces.

Thus, for example, the compound of formula (I) is a compound of formula (Ia) 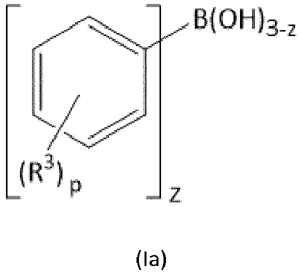 wherein R³ is as defined herein, and z is an integer selected from 1, 2 or 3.

For example, y and z can both be 1. However, the method of the invention does not necessarily yield a single compound of formula (I), but may result in a mixture of compounds of formula (I) having different numbers of phenyl substituents. That is, mixtures of compounds of formula (I) having different values of z.

For example, the main (or only) compound of formula (I) may be one wherein, when y is 1, z can also be 1 and R² is selected from the group consisting of -OH, halogen, C₁-C₄-alkyl, C₁-C₆-alkoxyl and C₆-C₁₀-aryloxyl, for example, wherein at least one of the two R² groups is a C₁-C₄-alkyl group. In another alternative, y is 0, the boron atom being tetrasubstituted and thus forming a borate salt wherein z can be 1 to 4, preferably 1 or 4.

Where a borate salt is formed (y is 0), the counter cation A can be any of the commonly used, for example, an alkali or earth alkaline metal cation or an ammonium cation, for example, an ammonium cation of formula +N(R^{a})(R^{b})(R^{c})(R^{d}), wherein each of R^{a}, R^{b}, R^{c} and R^{d} are independently selected from hydrogen and a C₁-C₆-alkyl optionally substituted with one or two hydroxyl groups. A is preferably an alkaline metal cation. The cation A has a charge a+ sufficient to neutralize the negative charge of the borate salt. Typically, a is 1 or 2, usually 1.

For example, in the main (or only) compound of formula (I) obtained y, z and R² can be any of the following combinations:
y is 1, z is 1 and both R² groups are -OH; or
y is 1, z is 1 and each R² is independently selected from the group consisting of halogen, C₁-C₄-alkyl, C₁-C₆-alkoxyl and C₆-C₁₀-aryloxyl; or
y is 1, z is 2, and R² is selected from the group consisting of -OH, halogen, C₁-C₄-alkyl, C₁-C₆-alkoxyl and C₆-C₁₀-aryloxyl; or
y is 1, z is 1 and both R² groups together form a bridging group -0-(CH₂)ᵣ-0-, wherein r is 2 or 3, so that the -0-(CH₂)ᵣ-0- group, together with the boron atom, form a 5- or 6-membered ring, where the CH₂ groups are optionally substituted by one or two C₁-C₄-alkyl groups.

Preferably, R³ is in each case fluoro or chloro. For example, R³ can be in each case fluoro or chloro, at position *para* or *meta* with respect to the boron atom.

The solvents used in the present reaction are those known for the Grignard reaction. The reaction can take place in polar aprotic solvents in the presence of the least possible amount of water.

Polar aprotic solvents are polar solvents without a functional group from which a proton can dissociate. The skilled person is aware of the different polar aprotic solvents available. Examples for suitable polar aprotic solvents are amides, such as N,N-dimethylformamide (DMF) and N,N-dimethylacetamide (DMA); sulfoxides, such as dimethylsulfoxide (DMSO); lactams, such as N-methylpyrrolidone (NMP); cyclic or non-cyclic ethers, such as tetrahydrofuran or methyltetrahydrofuran, 1,3-dioxane or 1,4-dioxane; ketones, such as acetone and methylethylketone; nitriles, such as acetonitrile; lactones, such as γ-butyrolactone; nitro compounds, such as nitromethane; ureas, such as tetramethyl urea or dimethylpropylene urea (DMPU); sulfones, such as sulfolan; and carbonic acid esters, such as dimethylcarbonate or ethylenecarbonate.

Preferred solvents in the reaction of the invention are ethers, either cyclic or non-cyclic. Typical examples are non-cyclic ethers, such as ethyl ether, diethoxymethane (DEM), cyclopentyl methyl ether (CPME), diglyme or t-butyl methyl ether (TBME), or cyclic ethers such as tetrahydrofuran (THF) or 2-methyl-THF (Me-THF) and mixtures thereof. It is preferred to use THF or Me-THF, more preferably Me-THF, which has many advantages, for example, a higher boiling point, it is safer to use (tends to form less peroxides), has a lower solubility in water (makes the work-up easier), it is more stable in the presence of strong acids and bases, and it is easy to recycle by simple distillation. It is also possible to use mixtures of solvents, such as mixtures of Me-THF with toluene or mixture of THF with toluene in weight proportions of 1:10 to 10:1, for example, 1:3 to 3:1.

### Preparation of the compounds of formula (V)

The compounds of formula (I) can be used to prepare agrochemical valuable products, for example, fluxapyroxad, pyraziflumid, bixafen or boscalid, or salts thereof. Thus, a further aspect is a process to prepare a compound selected from the group consisting of fluxapyroxad, pyraziflumid, bixafen, boscalid, and salts thereof, the process comprising preparing a compound of formula (I) by a process disclosed in the present application, and then transforming said compound of formula (I) into fluxapyroxad, pyraziflumid, bixafen, boscalid, or a salt thereof.

For example, the transformation of a compound of formula (I), or a mixture thereof, into a compound of formula (V) may comprise
coupling a compound of formula (I) with an aniline of formula (VI), or a salt thereof to form a compound of formula (VII) 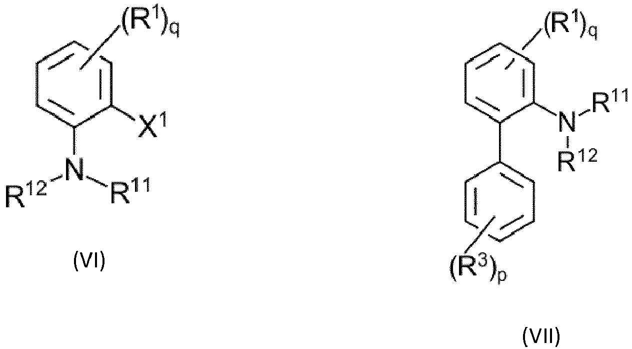 wherein
   q, R¹ and R³ are as defined elsewhere in the present document; and
   X¹ is a group capable of transmetalation with palladium;
   R¹¹ is as defined elsewhere in the present document;
   R¹² is selected from hydrogen and a nitrogen protecting group;
if required, obtaining a compound of formula (VII) wherein at least one of R¹¹ or R¹² is hydrogen;
   and
coupling the compound of formula (VII), or a salt thereof, wherein at least one of R¹¹ or R¹² is hydrogen, or a salt thereof, with a compound of formula (VIII)

   Q-C(=O)-Z formula (VIII)

   wherein
   Q is a defined above; and
   -C(=O)-Z is an acyl precursor.

This process is described in further detail in WO2021014437.

The reaction of the compounds of formula (I) with the compounds of formula (VI) in the presence of a palladium catalyst and a base provides the compounds of formula (VII), which are also key intermediates in the synthesis of active ingredients used in agriculture. It is preferred that in the compounds of formula (VI), q is 1 and R¹ is fluoro or that q is 0.

The condensation between a compound of formula (I) and an aniline of formula (VI) can take place in the presence of a palladium catalyst as described, for example, in WO2021014437 or in US2011105766.

It is preferred that p is 2, 3 or 4, and R³ is in each case fluoro or chloro. For example, it is preferred that p is 2 or 3. In a more preferred embodiment p is 2 or 3, R³ is in each case fluoro or chloro, q is 0 or 1, and R¹, if present, is fluoro or chloro. Further embodiments of the invention are
- p is 0 or 1, R³ is chloro, fluoro or -OH, q is 0 or 1, and R¹ fluoro or chloro or -OH;
- p is 2 or 3, R³ is in all cases fluoro, and q is 0; or
- p is 2 or 3, R³ is in all cases chloro, q is 0 or 1, and R¹ fluoro;
each leading to the compounds of formula (I) used as intermediates in the synthesis of boscalid, fluxapyroxad (or pyraziflumid) and bixafen, respectively.

The process may comprise the condensation of a compound to formula (VII) wherein at least one of R¹¹ or R¹² is hydrogen with a compound of formula (VIII) Q-C(=O)-Z, comprising the acyl precursor -C(=O)-Z. Acyl precursors that will provide the condensation with the amine group of the compound of formula (VII) are known to the skilled person. This reaction is specifically taught in many documents of the prior art, such as US 8,008,232 or WO 2018/035685 (see page 33).

For example, Z can be selected from the group consisting of -OH, halogen, -OSO₂R⁸, wherein R⁸ is C₁-C₁₆-alkyl, C₁-C₁₆-haloalkyl or C₆-C₁₆-aryl optionally substituted with a C₁-C₆-alkyl group, or a R^{a}COO- group wherein R^{a} is C₁-C₆ alkyl group, where possible, the groups being optionally substituted with one or more halogen atoms. Z is preferably halogen.

Compounds of formula (V) of interest are for example fluxapyroxad and bixafen, wherein Q is 3-(difluoromethyl)-1-methyl-pyrazole-4-carboxyl, or boscalid, wherein Q is 2-chloro-pyridine-3-carboxyl or pyraziflumid wherein Q is 3-trifluoromethylpyrazine-2-carboxyl. Thus, Q is preferably a C₃-C₈-heteroaryl, preferably comprising a 5 or 6 membered ring having 1 or 2 nitrogen atoms in the ring scaffold. It is thus preferred that Q is selected from the group consisting of pyridine, imidazole, pyrazole, pyrazine, pyrrol, furan, and thiophene, substituted with one or more residues selected from the group consisting of halogen, C₁-C₄-alkyl and C₁-C₄ haloalkyl.

The compounds of formula (VI) can be anilines substituted in the *ortho* position with an X¹ group, which is one capable of transmetalation with palladium. These groups are known in the art and are typically -Cl, -Br, -I or -OSO₂R⁸, wherein R⁸ is C₁-C₁₆-alkyl, C₁-C₁₆-haloalkyl or C₆-C₁₆-aryl optionally substituted with a C₁-C₆-alkyl group, for example, triflate (-OSO₂CF₃), mesylate (-OSO₂Me), tosylate (-OSO₂tolyl) or -OSO₂phenyl. It is preferable that X¹ is -Cl or -Br. Chlorine is often used when possible; however, it is sometimes necessary to use more reactive groups, such as bromine.

### EXAMPLES

### Synthesis of 3,4,5-trifluorophenylboronic acid: Grignard reagent over borate (Comparative)

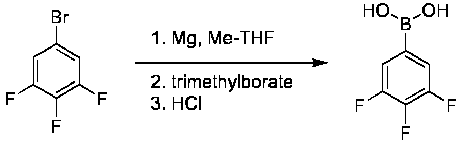

25.3 gr (1.05 mol, 1.1 eq) of Magnesium turnings were added to 200 ml Me-THF at RT under nitrogen. 200 gr (0.95 mol, 1.0 eq) of 3,4,5-trifluorobromobenzene in 400 ml Me-THF were placed in a dropping funnel. An initial portion of the 3,4,5-trifluorobromobenzene solution in Me-THF (60 ml) was added dropwise with stirring and the initiation of the Grignard reaction was awaited. Once initiated, a spontaneous temperature increase was detected, and turbidity of the reaction solution appeared. Subsequently, all the 3,4,5-trifluorobromobenzene solution was metered in over 3h. The mixture was stirred at 80°C for an additional 1h to complete the reaction, and then cooled to room temperature.

A 2^{nd} flask was initially charged with a solution of 118.4 mL (1.06 mol, 1.12eq) trimethyl borate and 200 ml Me-THF, which were precooled to 5°C. Thereafter, the Grignard solution was metered in, form the 1^{st} flask within 1.5h. the excess magnesium remained in the 1^{st} flask. Then, the mixture was stirred at RT for another 1h.

For hydrolysis: 80 ml of HCl were added dropwise at RT and the mixture was stirred for another 1h. The mixture was heated to 50°C and the phases were separated. The organic phase was washed with 100 ml water and the aqua phase was removed again. Subsequently, the organic solvent was evaporated to dryness and the obtained desired product (70% yield).

### Synthesis of 3,4,5-trifluorophenylboronic acid: Borate over Grignard reagent (according to the invention)

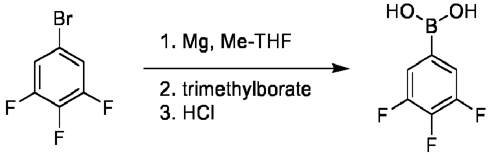

25.3 gr (1.05 mol, 1.1 eq) of Magnesium turnings were added to 200 ml Me-THF at RT under nitrogen. 200 gr (0.95 mol, 1.0 eq) of 3,4,5-trifluorobromobenzene in 400 ml Me-THF were placed in a dropping funnel. An initial portion of the 3,4,5-trifluorobromobenzene solution in Me-THF (60 ml) was added dropwise with stirring and the initiation of the Grignard reaction was awaited. Once initiated, a spontaneous temperature increase was detected, and turbidity of the reaction solution appeared. Subsequently, all the 3,4,5-trifluorobromobenzene solution was metered in over 3h. The mixture was stirred at 80°C for an additional 1h to complete the reaction, and then cooled to 5°C.

Afterwards, a solution of 118.4 mL (1.06 mol, 1.12eq) trimethyl borate and 200 ml Me-THF was metered in within 2h. Then, the mixture was stirred at RT for another 1h.

For hydrolysis: 80 ml of HCl were added dropwise at RT and the mixture was stirred for another 1h. The mixture was heated to 50°C and the phases were separated. The organic phase was washed with 100 ml water and the aqua phase was removed again. Subsequently, the organic solvent was evaporated to dryness and the obtained desired product (85% yield). Thus, the yield obtained in the process of the invention was increased from 70% to 85%.

## Claims

1. A process to prepare a compound of formula (I), or a mixture thereof 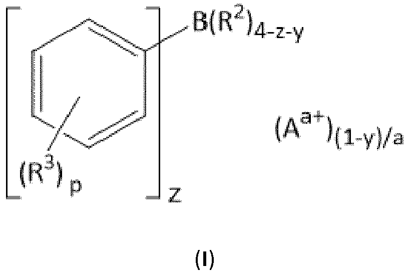 wherein
each R³ is independently selected from the group consisting of chloro, fluoro, C₁-C₆-alkyl, C₁-C₆-haloalkyl, C₁-C₆-alcoxyl and C₁-C₆-haloalcoxyl;
p is an integer selected from the group consisting of 1, 2, 3, 4 and 5
y is an integer selected from 0 or 1;
wherein,
when y is 1, then z is 1, 2 or 3;
when y is 0, then z is an integer selected from 1, 2, 3 or 4, and the compound of formula (I) forms a borate accompanied by a cation A having a charge a+;
and
each R² is independently selected from the group consisting of hydrogen, halogen, - OH, -OR⁹, and C₁-C₁₀-alkyl, wherein R⁹ is C₁-C₁₀-alkyl or C₆-C₁₂-aryl; or wherein, z being 1 or z being 2 and y being 0, two R² groups together form a bridging group -0-(CH₂)ᵣ-0-, wherein r is 2 or 3, so that said -0-(CH₂)ᵣ-0- group, together with the boron atom, form a 5- or 6-membered ring, where the CH₂ groups are optionally substituted by one or two C₁-C₄-alkyl groups;
the process comprising
a first step comprising the preparation of a compound of formula (II) by contacting a compound of formula (III) with a magnesium source 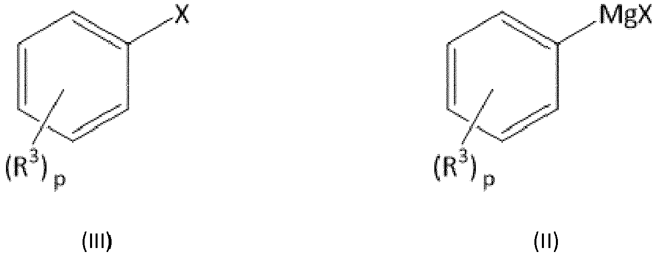 wherein R³ and p are as defined above, and X is a group capable of reacting with magnesium, selected from the group consisting of chloride, bromine, iodide and triflate; and
a second step comprising adding over the compound of formula (II) formed in the first step a compound of formula (IV)
B(R²)₃ (IV)
wherein R² is as defined above.

2. The process according to claim 1, wherein R³ is in each case fluoro or chloro.

3. The process according to any of the previous claims, wherein y is 1, z is 1 and R² is selected from the group consisting of -OH, halogen, C₁-C₄-alkyl, C₁-C₆-alkoxyl and C₆-C₁₀-aryloxyl.

4. The process according to any of the previous claims, comprising the step of hydrolyzing the compound of formula (I) in aqueous acid.

5. The process according to claims 1, 2 or 4, wherein the compound of formula (I) is a compound of formula (Ia) 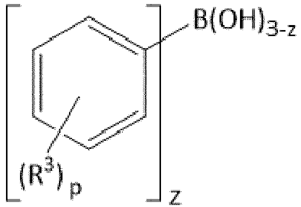 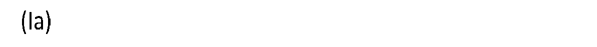 wherein R³ is as defined in any of the previous claims, and z is an integer selected from 1, 2 or 3.

6. The process according to claim 5, wherein z is 1.

7. The process according to any of the previous claims, wherein each R² in the compound of formula (IV) is independently selected from a C₁-C₁₀-alkyl group, preferably a C₁-C₄-alkyl group.

8. The process according to any of the previous claims, wherein the magnesium source and the compound of formula (III) are reacted at a temperature comprised between 20°C and 120°C.

9. The process according to any of the previous claims wherein the compound of formula (IV) is added over the compound of formula (II) at a temperature below 30°C.

10. A process to prepare a compound of formula (V), or a salt thereof 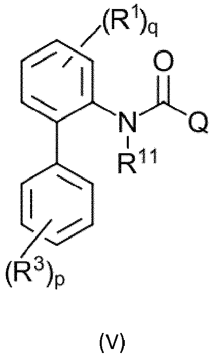 wherein
q is an integer selected from the group consisting of 0, 1, 2, 3 and 4;
p is an integer selected from the group consisting of 1, 2, 3, 4 and 5;
R¹¹ is selected from hydrogen or a nitrogen protecting group;
Q is C₆-C₁₅-aryl or C₃-C₁₅-heteroaryl, optionally substituted with one or more groups selected from the group consisting of halogen, C₁-C₄-alkyl and C₁-C₄-haloalkyl;
each R¹ is independently selected from the group consisting of halogen, -OH, C₁-C₆-alkyl, cyano, C₁-C₆-haloalkyl, C₁-C₆-alcoxyl, and C₁-C₆-haloalcoxyl; and
each R³ is independently selected from the group consisting of chloro, fluoro, C₁-C₆-alkyl, C₁-C₆-haloalkyl, C₁-C₆-alcoxyl and C₁-C₆-haloalcoxyl;
the process comprising
preparing a compound of formula (I) following the process as defined in any of the previous claims; and
then transforming said compound of formula (I) in a compound of formula (V).

11. The process according to claim 10 to prepare a compound selected from the group consisting of fluxapyroxad, pyraziflumid, bixafen, boscalid, and salts thereof, the process comprising preparing a compound of formula (I) by a process as defined in any of claim 1 to 9, and then transforming said compound of formula (I) in fluxapyroxad, pyraziflumid, bixafen, boscalid, or a salt thereof.
